# EUROPEAN PATENT APPLICATION

(11) **EP 0 691 352 A1**
(43) Date of publication of application: **10.01.1996**
(21) Application number: 95304178.7
(22) Date of filing: 16.06.1995
(51) Int. Cl.: C08F 8/14, C08J 3/12, C08J 3/24, C08F 8/32

(54) **Method of increasing the size and absorption under load of superabsorbent polymers by impregnation with a solution of cross-linking agent**

(30) Priority: 06.07.1994 GB 9413604; 30.05.1995 GB 9510900
(71) Applicant: AMCOL INTERNATIONAL CORPORATION, Arlington Heights, Illinois 60004 (US)
(72) Inventor: Henderson, John A., Birkenhead, L43 7RD (GB); Tomlin, Anthony S., Island Lake 60042, Illinois (US); Lucas, David M., West Kirby, L48 3LB, Merseyside (GB)
(74) Representative: W.P. Thompson & Co.

(57) **Abstract**

A method of enhancing the water absorbance of and increasing the particle size of fine water absorbent cross-linked polyacrylic polymer particles includes impregnating the cross-linked polyacrylic polymer particles with an impregnating solution containing a cross-linking agent for the polyacrylic polymer. The solution is impregnated into the polymer in a weight ratio of polymer, dry basis, to impregnating solution in the range of about 1:1 to about 1:10 and preferably at a weight ratio of polymer, dry basis, to impregnation water in the range of about 100:99 to about 100:999.9, and subjecting the impregnated polymer particles to conditions, such as increased temperature, e.g. 50°C - 150°C, to react the impregnated cross-linking agent within the polymer particles to form a further cross-linked polyacrylic polymer having enhanced capacity for absorption of fluids under load (AUL) and increased particle size.

## Description

### FIELD OF THE INVENTION

The present invention relates to a method of increasing the size and absorption under load of fine particles of a polyacrylic superabsorbent polymer (SAP) by intimately mixing the particles into a paste with an aqueous solution of a cross-linking agent. The paste then is subjected to conditions sufficient for cross-linking and drying, such as by subjecting the paste to extrusion and elevated temperature, so that the product is sufficiently cross-linked and dried for milling to a desired particle size range. The milled particles are larger in size than the original fine material (dry agglomeration). The so-formed agglomerates surprisingly remain as single particles upon hydration with saline (wet agglomeration) and display improved absorption under an applied load (AUL).

### BACKGROUND OF THE INVENTION AND PRIOR ART

Water-absorbing resins have found wide use in sanitary goods, hygenic goods, wiping cloths, water retaining agents, dehydrating agents, sludge coagulants, disposable towels and bath mats, disposable door mats, thickening agents, disposable litter mats for pets, condensation preventing agents, and release control agents for various chemicals. Water-absorbing resins are available in a variety of chemical forms including substituted and unsubstituted natural and synthetic polymers such as hydrolysis products of starch acrylonitrile graft polymers, carboxymethylcellulose, cross-linked polyacrylates, sulfonated polystyrenes, hydrolyzed polyacrylamides, polyvinyl alcohols, polyethylene oxides, polyvinylpyrrolidines and polyacrylonitriles.

In some polymerization processes for manufacturing water-absorbent polymers, such as cross-linked polyacrylic superabsorbent polymers, e.g., cross-linked polyacrylic acid or partially neutralized or fully neutralized cross-linked polyacrylic acid, one or more monomers are polymerized in water to produce a polymer that then must be ground to provide a desired particle size, with or without an intermediate drying step, for incorporation into a variety of different products, as outlined above. During the grinding process, fine particles result that are undesirable due to dusting, or other manufacturing problems. Fine superabsorbent material is considered to be undesirable in many personal care applications including infant diapers and adult incontinence devices. Such fine material can migrate in the device before use and exhibit gel blocking in application.

European Patent EP 0 463 388 A1 (Hoescht Celanese) discloses a process for recycling sub 75 µm SAP fines back into a reaction gel at 16% - 17% solids. However, only 4% recycle is possible and the addition of extra process water is required. International classification CO8L33/02 (Seitetsu) describes a method whereby fine powder may be blended with a prepared polyacrylate solution into a crumbly mix which generates agglomerates on drying at up to 150°C. International publication number WO 90/08789 (Dow) describes an agglomeration route involving the use of hydrocarbon solvents to suspend fine particles which are then clustered by the addition of acrylate monomer solution under polymerization conditions in the presence of an amorphous silica powder. Seitetsu also disclose the use of organic solvents as a dispersion phase in U.S. Patent No. 4,732,968 (EP 0 224 923). SAP fines are dispersed in an inert solvent with addition of silica in the presence of water and a suitable surfactant, followed by removal of solvent and drying.

All the above disclosures share disadvantages of either relatively low rates of fines consumption, or the use of large amounts of organic solvents and expensive silica additives. In addition there is no claim of enhanced superabsorbent performance other than lack of gel blocking in the agglomerated material.

U.S. Patent No. 4,950,692 discloses a method whereby SAP fines are wetted into a continuous amorphous gel by mixing with water in a suitable high shear mixer. The formed gel is dried and ground to a new particle size distribution. No claim was made for the properties of the new formed on-size SAP product.

In the present invention, a process is disclosed wherein fine SAP particles are impregnated with an aqueous solution of cross-linking agent during mixing, e.g., high shear mixing, such as in a Z-blade mixer or Sigma blender, in order to generate a continuous high viscosity paste. The paste is subjected to conditions which simultaneously promote drying of the product and reaction of the cross-linking agent. Upon subsequent milling of the dry product, particles of increased particle size are obtained. These particles retain particle integrity upon hydration and demonstrate enhanced polymer performance in the absorption under load test.

### SUMMARY OF THE INVENTION

In brief, the present invention is directed to a process for increasing the size of fine particles of a superabsorbent polymer (SAP) selected from cross-linked polyacrylic acid polymers, cross-linked polyacrylate polymers (partially or fully neutralized polyacrylic acid polymers), and mixtures thereof, and, more particularly, to a process of impregnating the SAP particles with a cross-linking agent for polyacrylic acid and/or metal salts of polyacrylic acid. Impregnation is achieved by intimately mixing the polymer, in the form of polymer fines (e.g., having a particle size below about 300 µm) with an aqueous solution of the crosslinking agent to form a continuous paste, e.g., having a viscosity in the range of about 20,000 to about 5,000,000 centipoise. Cross-linking within, and drying of, the paste then is preferably achieved by heat. The dry product (0 to about 15% by weight water based on the dry weight of the polymer particles) is then milled to a suitable particle size distribution, to yield a product of improved particle size distribution (hereinafter defined as dry agglomeration), which retains its agglomerated nature upon hydration (hereinafter defined as Wet Agglomeration) and displays improved absorption under load (AUL), described in more detail hereinafter, when compared to the original fine material.

In accordance with the present invention, the fine particles of SAP are impregnated with an aqueous solution of cross-linking agent for the SAP. The solution preferably contains from about 0.01% to 1% w/w cross-linking agent, more preferably 0.03% to 0.1% cross-linking agent with the remainder, preferably, being water. The solution is impregnated into the particles at a weight ratio of polymer to solution in the range of about 1:1 to about 1:10, preferably about 1:1 to about 1:5, and more preferably about 1:1 to about 1:3, in a suitable high shear mixer to form a continuous paste. The ratio of cross-linked polymer to impregnated water is also preferably in the ratio of about 1:1 to about 1:10 since the cross-linking agent is present in a concentration of only about 0.01% to 1% by weight of the aqueous solution of cross-linking agent impregnating the solid SAP particles. The mixture of fines, cross-linking agent, and water has the consistency of a relatively stiff dough, and the mixing is sufficiently thorough that the particle memory of the original fines is lost and the cross-linking agent-containing solution becomes thoroughly impregnated into the fine SAP particles. Suitable mixers include Z-blade or Sigma blade mixers, planetary mixers, or other mixers designed to process high viscosity mixes.

The formed paste is conveniently rendered suitable for efficient drying/cross-linking by extrusion into strands, which are then subject to sufficient temperature, in the range of about 50°C to about 150°C, preferably about 120°C to about 150°C, such that the impregnated cross-linking agent is activated and the polymer is sufficiently cross-linked and sufficiently dry and brittle to mill or otherwise pulverize into a desired particle size distribution.

To achieve the full advantage of the present invention the combination of impregnating solution and fines should be of a consistency to retain a hand molded shape or retain a strand structure upon extrusion. It should not be too soft to prevent efficient processing, nor so stiff as to damage the product through excessive shearing during mixing.

Accordingly, one aspect of the present invention is to provide a process for increasing the size of cross-linked SAP particles by impregnating the particles with an aqueous solution of cross-linking agent, and activating the cross-linking agent, to further cross-link the SAP particles and/or cross-link adjacent resin particles together, thereby increasing the size of the SAP particles and increasing their water-absorbing performance.

Still another aspect of the present invention is to provide a process for increasing the size of fine, cross-linked water-absorbent SAP resin particles by impregnating the particles with an aqueous solution of cross-linking agent and activating the cross-linking agent, to further crosslink the resin particles and/or to cross-link adjacent resin particles together to form particle agglomerates, thereby increasing the size of the resin particles and increasing their water-absorbing performance. The ratio of the weight of solid SAP particles to the weight of impregnating solution should be in the range of about 1:1 to about 1:10, preferably about 1:1 to about 1:5, more preferably about 1:1 to about 1:3. Since in the preferred embodiment, the aqueous impregnating solution contains about 0.01% to 1% cross-linking agent, the weight ratio of solid SAP particles to water is preferably in the ratio of 100:99 to 100:999.9.

Another aspect of the present invention is to provide water-absorbent acrylic SAP resin particles having increased size, and/or increased absorption under load, wherein absorbed liquid, e.g., water or urine, is more difficult to squeeze out of the SAP resin particles.

Another aspect of the present invention is to provide a process of producing cross-linked polyacrylic SAP resin particles of increased size, by impregnating solid, cross-linked polyacrylic polymer (SAP) particles with an aqueous solution containing a cross-linking agent for the SAP polymer, to form an impregnated acrylic (SAP) polymer having a dough or paste consistency, and then subjecting the impregnated SAP polymer paste to conditions sufficient to activate the cross-linking agent, thereby increasing the size of the SAP particles.

The above and other aspects and advantages of the present invention will become more apparent from the following detailed description of the preferred embodiments.

### DETAILED DESCRIPTION OF THE PREFERRED EMBODIMENTS

Fine particles of water-absorbing, cross-linked acrylic resin are increased in size and water-absorbing capability by impregnating relatively fine particles of SAP with an aqueous solution containing a crosslinking agent, and subjecting the impregnated SAP polymer particles to conditions sufficient to activate the impregnated cross-linking agent to further cross-link the polymer molecules, or to cross-link adjacent molecules of SAP polymer.

The solid SAP particle fines preferably have a particle size less than about 300 microns (µm). The fine particles of acrylic, water-absorbent resin (SAP) can be initially manufactured by any process and such fine acrylic particles can have their size increased in accordance with the method of the present invention. The resulting, increased size polymer particles display wet agglomeration and improved absorption under load (AUL) upon hydration.

In accordance with an important embodiment of the present invention, fine particles of a cross-linked SAP are impregnated with a solution containing a cross-linking agent for the SAP polymer particles, such that the impregnated particles have a water content of at least about 20%, preferably at least about 40% by weight water. The impregnated solid particles are thoroughly wetted with the aqueous solution of crosslinking agent by combining the solid resin particles with the solution in a weight ratio of solid resin particle (dry polymer basis) to solution in the range of about 1:1 to about 1:10, preferably about 1:1 to about 1:5, and more preferably about 1:1 to about 1:3; and preferably a weight ratio of solid resin particles to impregnating water from the aqueous solution containing crosslinking agent in the range of about 100:99 to 100.999.9.

In accordance with the preferred embodiment of the present invention, improved SAP particles, having increased particle size (dry agglomeration), retention of particle integrity upon hydration (wet agglomeration) and increased absorption under load are prepared by impregnating fine solid particles of a water-insoluble, cross-linked, water-absorbent acrylic polymer with an aqueous solution containing a crosslinking agent in an amount in the range of about 0.005% to about 1% by weight, preferably about 0.01% to about 0.3%, more preferably about 0.03% to about 0.1% by weight cross-linking agent, based on the total weight of the aqueous solution. After impregnating the solid resin particles, and thoroughly and homogeneously mixing the cross-linking agent-containing solution with the solid resin particles to a consistency of a dough or paste, the mix is subjected to conditions sufficient to activate the cross-linking agent, e.g., 120oC, for a time sufficient that the mass of impregnated resin particles are sufficiently dry to mill or pulverize the dough to a desired particle size distribution (about 15% by weight, or less, water).

Suitable cross-linking agents for the purpose of agglomeration by cross-linking agent impregnation are di- or poly-functional molecules, or any cross-linking agent capable of cross-linking polyacrylic acid and/or metals salts of polyacrylic acid by reaction with the acrylic or acrylate functional groups of the polymer. Such cross-linking agents include diglycidyl ethers, dialcohols, and diamines. Preferably the cross-linking agent should be water-soluble and possess reactivity with the polyacrylic polymer such that cross-linking occurs in a controlled fashion in the temperature range of about 50°C to about 150°C. Examples of suitable cross-linking agents include ethylene glycol, polyethylene glycols, polypropylene glycols, and diglycidyl ethers of (poly) ethylene glycols. Of particular preference is ethylene glycol diglycidyl ether (EGDGE), a water-soluble diglycidyl ether which cures (cross-links) polyacrylic acid and polyacrylate (SAP) molecules within the preferred 50°C - 150°C temperature range.

The weight ratio of resin particles to cross-linking agent-containing solution should be in the range of about 1:1 to about 1:10, preferably about 1:1 to about 1:5, and more preferably about 1:1 to about 1:3, to provide impregnated resin particles having a doughy or pasty consistency that is easily processed without excessive shearing during mixing. Since the concentration of cross-linking agent in the SAP particle impregnating solution is preferably in the range of about 0.01% to about 1% by weight, the weight ratio of SAP particles to impregnation water is preferably in the range of about 100:99 to about 100:999.9. The resin particles, prior to impregnation, preferably have a particle size less than about 300 µm. The cross-linking reaction of the cross-linking agent with the impregnated particles enlarges the particles and improves their water-absorbency properties. It is understood, however, that the process of the present invention also is useful to improve the water-absorbency properties of cross-linked polyacrylic polymers regardless of their particle size. Activation of cross-linking agent that is thoroughly impregnated into the SAP fines decreases the number of particles and increases the size of the particles, without particle number increase upon hydration (wet agglomeration), and enhances their absorption under load.

The cross-linking agent solution can be prepared easily by adding the cross-linking agent to water, and the solution then is thoroughly mixed with and impregnated into the resin particles, for example in a Z-blade mixer or Sigma blender.

The cross-linking reaction is activated in any manner after impregnation of the particles, e.g., by raising the temperature of the impregnated particles to a temperature of about 50°C to about 150°C, preferably at least about 120°C, e.g., about 120°C to about 150°C. It is preferred that the impregnated dough is subdivided into a smaller mass, e.g., by extrusion of the dough through die openings of about 4 mm. to about 6 mm. in diameter, prior to activating the cross-linking agent. After mixing the cross-linking agent into solution, and after thoroughly and homogeneously mixing and impregnating the resin particles with the solution, the cross-linking reaction proceeds rapidly and controllably, for example, by heating the dough of impregnated resin particles to a temperature sufficient to activate the cross-linking agent.

This cross-linking reaction, of the cross-linking agent contained in the solution that is impregnated into the resin particles, provides additional cross-linking of the resin particles not only on an outer surface of the fine particles, but also within the interior of the fines to provide larger resin agglomerate particles that do not separate upon hydration. Subsequently, after a drying step, if needed, the solid crosslinked polymer agglomerates can be made into a desired particle size distribution, by the usual method, for example, by milling or pulverizing. Any resulting fines can be treated again in accordance with the method of the present invention so that there are essentially no wasted fine particles.

### Dry Agglomeration

Dry agglomeration is defined as a change in particle size distribution of a dry agglomerated powder fraction towards larger particle sizes than the original fines powder fraction. Dry agglomeration was determined by shaking samples over a standard, 300 µm mesh, screen (U.S. Sieve No. 50) and measuring the percentage retention on the screen. Fine SAP particles used to prepare agglomerates were sized to less than 300 µm (U.S. Sieve No. 50) before use.

### Wet Agglomeration

Wet agglomeration is defined as the ability of an SAP agglomerate to retain its single particle nature upon hydration, i.e., a lack of deagglomeration upon hydration. Wet agglomeration was determined by weighing out 50 agglomerate particles on a watch glass and hydrating them with 20 times their weight in 1% by weight sodium chloride solution (1% saline). The particles were allowed to absorb the saline solution for one hour and then the number of particles was recounted under a microscope.

### Absorption Under Load

Absorption under load (AUL) is a measure of the ability of a superabsorbent polymer to absorb fluid under an applied pressure. The AUL was determined by the following method as disclosed in European Patent No. 443,627 and corresponding U.S. Patent No. 5,149,335, hereby incorporated by reference.

0.160 g +/-0.001 g of SAP is carefully scattered onto a 140 µm water-permeable mesh attached to the base of a hollow plexiglass cylinder having an internal diameter of 25 mm. The sample is covered with a 100 g cover plate and the cylinder assembly weighed. This gives an applied pressure of 20 g/cm. The screened base of the cylinder is placed in a 100 mm petri dish containing 25 mls of 1 % by weight saline, and the polymer is allowed to absorb the saline solution for 1 hour. By reweighing the cylinder assembly, the AUL may be calculated by dividing the weight of liquid absorbed by the dry weight of polymer before liquid contact.

The present invention is described in greater detail with reference to the following examples:

### Example 1

To 1.5 kg of a 0.01% by weight solution of ethylene glycol diglycidyl ether (DENACOL 810, Nagase Chemical Company) in the chamber of a Winkworth Model 14Z Z-blade mixer/extruder (Winkworth Ltd., Reading, UK), was gradually added 1.5 kg of superabsorbent polymer (sodium polyacrylate) fines (particle size less than 300 µm). Mixing was extended for five minutes until the polymer and liquid were thoroughly mixed. The resultant high viscosity (2,000,000 centipoise) paste was extruded through a mincer head die plate, having 4 mm apertures, and laid flat upon perforated trays. The extrudate was dried for one hour at 120°C in a forced air oven, until sufficiently brittle to mill (around 10% by weight water).

The dry and wet agglomeration characteristics of the chosen on size (850 µm - 300 µm) fraction of the resultant powder and its AUL are summarized in Table 1. The conditions of Example 1 were subject to the variations summarized in Table 1 with the tabulated effect on dry/wet agglomeration and AUL.

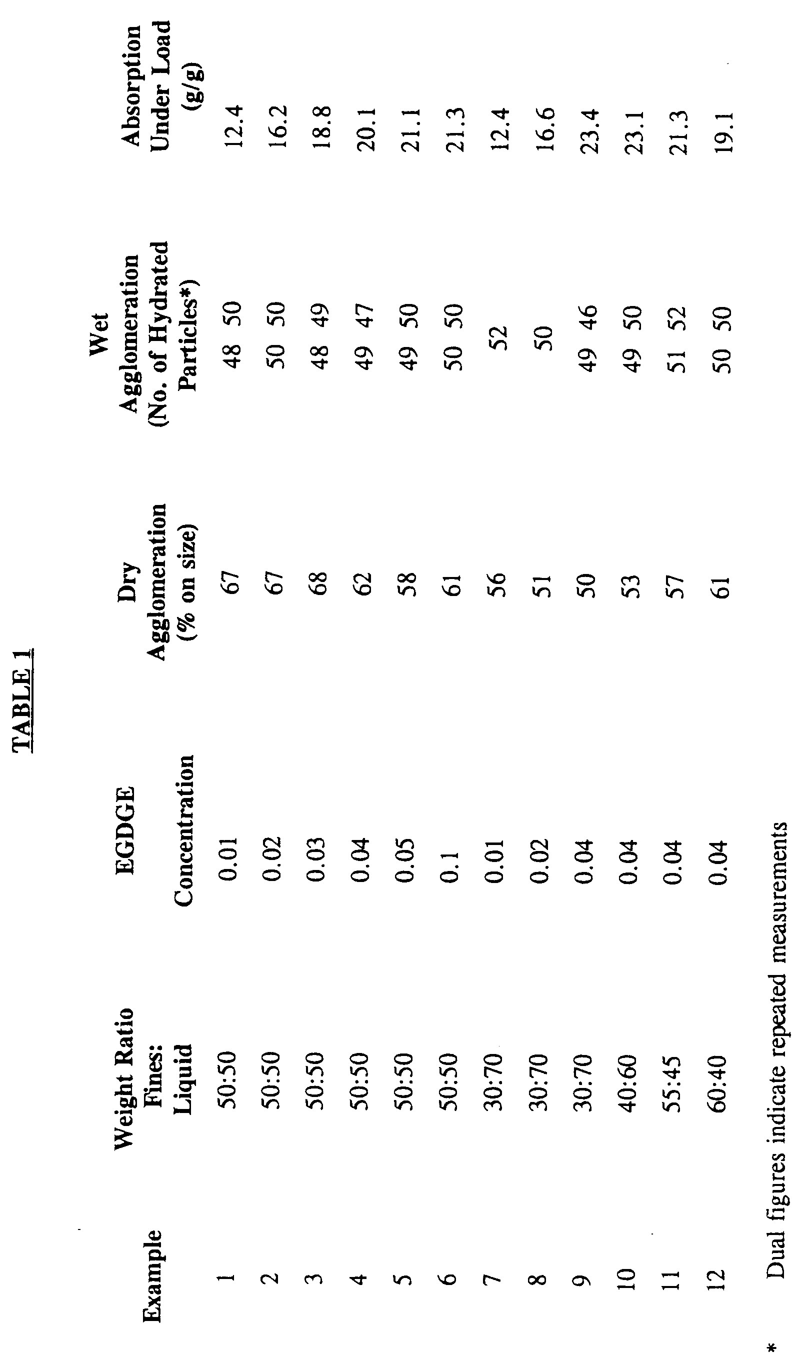

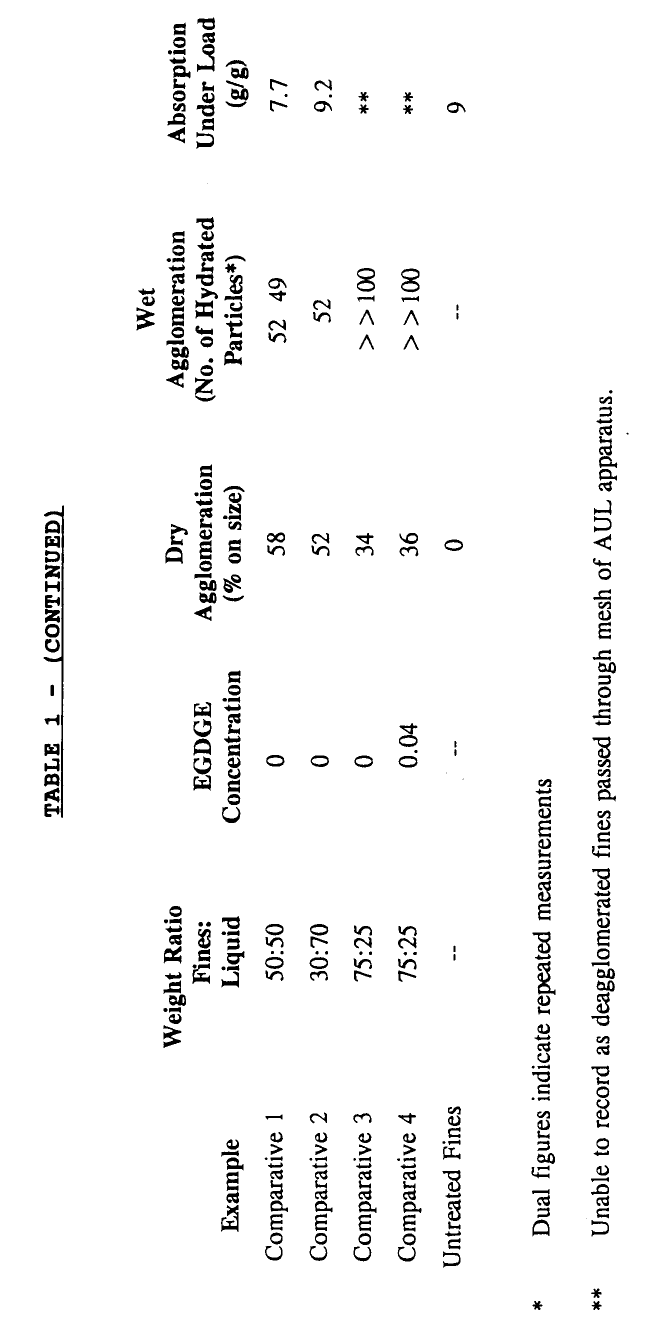

It will be understood that the present disclosure has been made only by way of preferred embodiments and that numerous changes in details of construction, combination, and arrangement of parts can be resorted to without departing from the spirit and scope of the invention as hereunder claimed.

## Claims

1. A method of enhancing the water or aqueous medium absorbance and particle size of water-absorbent, cross-linked polyacrylic polymer particles comprising:
impregnating the cross-linked polyacrylic polymer particles with an impregnating solution containing a cross-linking agent for the polyacrylic polymer;
wherein the solution is impregnated into the cross-linked polyacrylic polymer in a weight ratio of polyacrylic polymer, dry basis, to impregnating solution in the range of about 1:1 to about 1:10, and
subjecting the impregnated polyacrylic polymer to conditions sufficient to react the impregnated crosslinking agent within the polyacrylic polymer particles to form a further cross-linked polyacrylic polymer having enhanced water absorbance.

2. A method as claimed in claim 1, wherein the weight ratio of polyacrylic polymer to impregnating solution is in the range of about 1:1 to about 1:5.

3. A method as claimed in claim 2, wherein the weight ratio of polyacrylic polymer to impregnating solution is in the range of about 1:1 to about 1:3.

4. A method as claimed in any one of the preceding claims, wherein the impregnated polyacrylic polymer is heated to a temperature sufficient to further cross-link the polyacrylic polymer.

5. A method as claimed in claim 4, wherein the impregnated polyacrylic polymer is heated to a temperature of at least about 50°C for a time sufficient to form a polyacrylic polymer sufficiently brittle to be pulverized.

6. A method as claimed in claim 5, wherein the impregnated polyacrylic polymer is heated to a temperature of about 50°C to about 150°C.

7. A method as claimed in claim 6, wherein the impregnated polyacrylic polymer is heated to a temperature of about 100°c to about 150°C.

8. A method as claimed in claim 7, wherein the impregnated polyacrylic polymer is heated to a temperature of about 120°C to about 150°C.

9. A method as claimed in claim 7, wherein the impregnated polyacrylic polymer is heated to a temperature of about 110°C to about 130°C.

10. A method as claimed in any one of the preceding claims, wherein the polyacrylic polymer particles have a size less than about 300 µm, prior to impregnation.

11. A method as claimed in any one of the preceding claims, wherein the polyacrylic polymer particles have a size less than about 250 µm, prior to impregnation.

12. A method as claimed in any one of the preceding claims, further including the step of pulverizing the polyacrylic polymer to a desired particle size distribution, after reaction of the cross-linking agent within the polyacrylic polymer particles.

13. A method as claimed in claim 12 further including the steps of
collecting cross-linked polyacrylic polymer particles resulting from the pulverizing step that have a size below about 300 µm,
impregnating the collected polyacrylic polymer particles with an impregnating solution containing a cross-linking agent for the polyacrylic polymer;
wherein the solution is impregnated into the polyacrylic polymer in a weight ratio of polyacrylic polymer, dry basis, to impregnating solution in the range of about 1:1 to about 1:10, and
subjecting the impregnated polyacrylic polymer to conditions sufficient to react the impregnated crosslinking agent within the polyacrylic polymer particles to form a further cross-linked polyacrylic polymer having an increased particle size.

14. A method as claimed in any one of the preceding claims, wherein the solution of cross-linking agent comprises about 0.005% to about 1% by weight cross-linking agent, based on the total weight of the solution of cross-linking agent.

15. A method as claimed in claim 14, wherein the solution of cross-linking agent comprises about 0.01% to about 0.3% by weight cross-linking agent.

16. A method as claimed in claim 15, wherein the solution of cross-linking agent comprises about 0.03% to about 0.1% by weight cross-linking agent.

17. A method as claimed in any one of the preceding claims. wherein the solution of crosslinking agent is an aqueous solution.

18. A method as claimed in claim 17, wherein the solution of cross-linking agent comprises the crosslinking agent dissolved in a carrier consisting essentially of water.

19. A method as claimed in any one of the preceding claims, wherein the cross-linking agent is selected from the group consisting of ethylene glycol diglycidyl ether, polyglycidyl ether, polyols, polyamines and mixtures thereof.

20. A method as claimed in any one of the preceding claims, wherein the cross-linking agent is a diglycidyl ether.

21. A method as claimed in any one of the preceding claims, wherein the impregnated polyacrylic polymer is heated to a temperature sufficient to further cross-link molecules of the impregnated polyacrylic polymer, or to cross-link adjacent polyacrylic polymer molecules, and to enlarge the polyacrylic polymer particles.

22. A method as claimed in any one of the preceding claims, wherein the cross-linking agent is contained in the impregnating solution in an amount of about 0.01% to about 0.5% by weight, based on the total weight of impregnating solution.

23. A method as claimed in any one of the preceding claims, wherein the cross-linking agent is selected from the group consisting of ethylene glycol diglycidyl ether, polyglycidyl ethers, polyols, polyamines and mixtures thereof.

24. A method of increasing the size and water absorbance of water-absorbent cross-linked polyacrylic polymer particles, having a size, prior to treatment, predominantly less than about 300 µm comprising:
impregnating the polyacrylic polymer particles with an impregnating solution containing a crosslinking agent for the polymer;
wherein the solution is impregnated into the polymer particles in a weight ratio of polyacrylic polymer, dry basis, to impregnating solution in the range of about 1:1 to about 1:10, and
subjecting the impregnated polymer particles to conditions sufficient to further cross-link the impregnated polyacrylic polymer particles to form polyacrylic polymer particles having increased size.

25. A method as claimed in claim 24, wherein the polyacrylic polymer particles have an initial size less than about 250 µm, prior to impregnation.

26. A method as claimed in any one of the preceding claims, further including the step of pulverizing the polyacrylic polymer particles to a desired particle size distribution after further cross-linking of the polyacrylic polymer particles with the impregnated solution of cross-linking agent.

27. A method as claimed in claim 26, wherein after pulverizing the cross-linked polyacrylic polymer particles, the particles do not separate into smaller particles upon hydration.

28. A method as claimed in any one of the preceding claims, wherein the polyacrylic polymer is polyacrylic acid neutralized at least 25 mole percent.

29. A method as claimed in any one of the preceding claims, wherein the polyacrylic polymer is polyacrylic acid neutralized 50 to 90 mole percent.

30. A method as claimed in claim 29, wherein the polyacrylic polymer is polyacrylic acid neutralized 60 to 80 mole percent.

31. A method as claimed in any one of the preceding claims, wherein the solution is impregnated into the cross-linked polyacrylic acid in the weight ratio of polyacrylic polymer, dry basis, to impregnation water in the range of about 100:99 to about 100:999.9

32. Cross-linked polymer particles whenever produced by a process as claimed in any one of the preceding claims.
